**Europäisches Patentamt**

**European Patent Office**

**Office européen des brevets**

(11) Veröffentlichungsnummer: **0 457 135 A2**

# (12) EUROPÄISCHE PATENTANMELDUNG

(21) Anmeldenummer: **91107234.6**

(22) Anmeldetag: **03.05.91**

(51) Int. Cl.5: **A61M 5/20**

(30) Priorität: **12.05.90 DE 9005419 U**

(43) Veröffentlichungstag der Anmeldung:
**21.11.91 Patentblatt 91/47**

(84) Benannte Vertragsstaaten:
**CH DE FR LI**

(71) Anmelder: **Nothdurft, Klaus**
**Vaihinger Strasse 50**
**W-7000 Stuttgart 80(DE)**

(72) Erfinder: **Nothdurft, Klaus**
**Vaihinger Strasse 50**
**W-7000 Stuttgart 80(DE)**

(74) Vertreter: **Schmid, Berthold**
**Kohler Schmid + Partner Patentanwälte**
**Ruppmannstrasse 27**
**W-7000 Stuttgart 80(DE)**

(54) **Betätigungsvorrichtung für eine Einmalspritze.**

(57) Um eine kleine, leichte und problemlos zu handhabende und insbesondere für Sehbehinderte einfach benutzbare Vorrichtung zum Injizieren eines Mittels aus einer Einmalspritze zu bekommen, ist am Vorrichtungsgehäuse (1) eine Gehäuseverlängerung (2) mittels eines Bajonettverschlusses (3) gehalten. In der sogenannten Transportstellung kann man die Gehäuseverlängerung (2) in das Vorrichtungsgehäuse (1) einschieben, so daß die Gesamtlänge durch die Länge des Vorrichtungsgehäuses (1) allein bestimmt ist.

Bei der Benutzung befindet sich die Einmalspritze (11) im wesentlichen in der Gehäuseverlängerung (2), jedoch ragt sie teilweise auch in das bajonettverschlußseitige Ende des Vorrichtungsgehäuses (1) hinein. Dort befinden sich zwei Durchtrittsfenster (15), durch welche die Grifflappen (14) der Spritze (11) nach außen vorstehen.

Die das eigentliche Spritzen bewirkende Belastungsfeder (8) für den Vorrichtungskolben (9) wird mit Hilfe der Einmalspritze gespannt, indem man diese gewissermaßen in umgekehrtem Sinne in das Vorrichtungsgehäuse (1) hineindrückt, wobei allerdings eine Schutzkappe die Nadel noch umhüllt. Mit Hilfe der Schutzkappe wird der Kolben (9) in die Verraststellung verschoben und gleichzeitig die Belastungsfeder (8) gespannt.

Fig.7

Fig.3

EP 0 457 135 A2

Die Erfindung bezieht sich auf eine Betätigungsvorrichtung für eine mit einer Nadelschutzkappe sowie zwei Grifflappen versehene Einmalspritze, mit einem federbelasteten, in einer gespannten Verschiebestellung verrastbaren, mittels eines Betätigungsorgangs auslösbaren, in einem Vorrichtungsgehäuse verschiebbaren Vorrichtungskolben mit einem bewegbaren Verrastelement, das in der gespannten Verschiebestellung mit einem gehäusefesten Verrast-Gegenelement zusammenwirkt und mit einer abnehmbaren hülsenartigen Gehäuseverlängerung. Solche und ähnliche Betätigungsvorrichtungen für Einmalspritzen gibt es in den verschiedensten Ausführungen. Sie sind für die unmittelbare Benutzung durch den Patienten selbst gedacht. Er injiziert sich mit Hilfe der Einmalspritze ein verordnetes Medikament, beispielsweise Insulin. Zu diese Zwecke füllt er die Einmalspritze mit der vorgeschriebenen Menge, bringt sie anschließend in die Betätigungsvorrichtung und löst letztere daraufhin mit Hilfe des Betätigungsorgans aus. Der zuvor zurückverschobene und dadurch unter starker Federbelastung stehende Kolben trifft schlagartig auf der Kolbenstange des Kolbens der Einmalspritze auf. Die Nadel steht zu Beginn des Auslösevorgangs über die Vorrichtung bzw. das Aufsetzende der Gehäuseverlängerung nicht über. Beim Auftreffen des Vorrichtungskolbens auf der Kolbenstange der Einmalspritze wird zunächst letztere in der Vorrichtung so weit verschoben, daß die Nadel weit genug über die Betätigungsvorrichtung vorsteht. Sie dringt dabei unmittelbar in die Hautpartie auf, auf welcher die Betätigungsvorrichtung aufgesetzt worden ist. Dieses Einstechen ist dadurch möglich, daß die Flüssigkeit im Inneren der Einmalspritze inkompressibel ist und sie nicht schlagartig über die Nadel austreten kann. Erst nachdem die Nadel in den Körper eingedrungen ist, wird der Spritzenkolben im Spritzenzylinder langsam nach vorne geschoben und dadurch das Ausspritzen des Medikaments in den Körper des Patienten bewirkt.

Es ist einleuchtend, daß die Betätigungsvorrichtung vom Patienten, insbesondere von einem sehschwachen oder gar blinden Patienten problemlos bedienbar sein muß. Außerdem sollte sie möglichst klein sein, damit sie gut zu handhaben und zu transportieren ist. Das Spannen der Belastungsfeder für den Kolben, das Auslösen des letzteren, das Einlegen der Spritze und das Herausnehmen nach der Benutzung sollte so einfach wie möglich vonstatten gehen.

Um die Einmalspritze ins Innere der Betätigungsvorrichtung bringen zu können, ist diese zweiteilig ausgebildet. Sie besitzt ein Vorrichtungsgehäuse mit dem Auslösemechanismus sowie eine Gehäuseverlängerung, wobei beide Teile auf möglichst einfache Weise rasch verbindbar und lösbar sein müssen. Wenn die Gehäuseverlängerung vom Gehäuse abgenommen ist, kann die das Spritzmedium enthaltene Spritze nach vorherigem Spannen des Auslösemechanismus eingelegt werden. Vor dem Auslösen darf, wie gesagt, die Nadel über das freie Ende der Gehäuseverlängerung nicht überstehen.

Es liegt nun die Aufgabe vor, diese Betätigungsvorrichtung so weiterzubilden, daß sie auf einfachste Weise gespannt, die Spritze problemlos eingelegt und anschließend die Gehäuseverlängerung auf unkomplizierte Weise mit dem Gehäuse verbunden werden kann, und zwar sowohl für den Transport als auch das Spritzen.

Zur Lösung dieser Aufgabe wird erfindungsgemäß vorgeschlagen, daß die Bestätigungsvorrichtung gemäß dem Oberbegriff des Anspruchs 1 entsprechend dem kennzeichnenden Teil dieses Anspruchs ausgebildet ist. Bei dieser Vorrichtung macht man sich den Umstand nutzbar, daß die Nadel jeder Einmalspritze von einer Kappe überdeckt ist, die fest und unverschiebbar am Spritzenzylinder gehalten ist. Diese Nadelschutzkappe steckt man in das Ende des Vorrichtungsgehäuses ein, das anschließend mit der Gehäuseverlängerung verbunden werden soll. Man drückt die Nadelschutzkappe gegen den Vorrichtungskolben und verschiebt dadurch letzteren gegen den Widerstand der Belastungsfeder gegen das Auslöseorgan hin. Am Ende dieser Verschiebebewegung findet ein Verrasten des bewegbaren Verrastelements am Vorrichtungskolben mit dem gehäusefesten Verrast-Gegenglied der Vorrichtung statt. Dieses Verschieben des Vorrichtungskolbens ist dadurch leicht möglich, daß die Höhlung des Vorrichtungszylinders dem Durchmesser der Nadelschutzkappe bzw. dem größten Nadelschutzkappendurchmesser entsprechend dimensioniert ist. Außerdem sind die Endstellung des verschobenen Vorrichtungskolbens und die Länge des Vorrichtungsgehäuses vom zurückgeschobenen Kolben bis zum Einsteckende für die Spritze mit der Nadelschutzkappe so zu wählen, daß das Spannen durchgeführt werden kann, bevor die Grifflappen der Spritze am Vorrichtungsgehäuse aufgetroffen sind.

Falls die Durchtrittsfenster für die Grifflappen im Vorrichtungsgehäuse ausgebildet sind, kann man sie aufgrund ihrer axialen randoffenen Ausbildung in der Endphase des Spannens der Kolbenfeder ausnutzen, indem man die Grifflappen wenigstens teilweise in die Durchtrittsfenster einschiebt. Um aber die Vorrichtung möglichst blindlings bedienen zu können, ist es empfehlenswert, auf das Einführen der Spritzenlappen in die Durchtrittsfenster zu verzichten und die Gehäusedimensionierung so zu wählen, daß die Grifflappen bei gespanntem Kolben das zugeordnete freie Ende des Vorrichtungsgehäuses überhaupt nicht erreichen.

Nach dem Spannen des Kolbens nimmt man die Spritze wieder heraus, zieht die Nadelschutzkappe ab und füllt die Spritze. Daraufhin steckt man sie mit der Nadel voran in die Gehäuseverlängerung ein und verbindet letztere anschließend mit dem Vorrichtungsgehäuse. Zuvor oder bei dieser Gelegenheit werden die Grifflappen der Spritze in die randoffenen Enden der Durchtrittsfenster eingeführt. Beim Verbinden des Vorrichtungsgehäuses mit der Gehäuseverlängerung werden diese in Achsrichtung randoffenen Enden der Durchtrittsfenster durch das jeweils andere Gehäuseteil verschlossen. Über die Gehäuselappen kann man die gesamte Spritze in der Vorrichtung erforderlichenfalls so weit verschieben, daß die Nadel über die Mündung der Gehäuseverlängerung nicht vorsteht. In dieser Stellung liegt die Kolbenstange der Einmalspritze im Normalfalle am zurückgezogenen Vorrichtungskolben noch nicht an.

Man setzt nunmehr die Spritze an der vorgesehenen Stelle, beispielsweise dem Bauch oder dem Bein des Patienten an, wirkt auf das Betätigungsorgan ein und löst dadurch den Arbeitshub des Vorrichtungskolbens aus. Das Einwirken auf das Betätigungsorgan bewirkt das Lösen der Verrastvorrichtung.

Anschließend nimmt man die Gehäuseverlängerung ab und entfernt die ausgebrauchte Einmalspritze.

Weitere vorteilhafte Ausgestaltungen dieser Vorrichtung und die hieraus resultierenden Vorteile ergeben sich aus der nachfolgenden Beschreibung eines Ausführungsbeispiels. Die Zeichnung zeigt ein solches Ausführungsbeispiel. Hierbei stellen dar:

Fig. 1    Eine Seitenansicht der Vorrichtung in der Transport- oder Aufbewahrungsstellung,

Fig. 2    eine Seitenansicht der auslösebereiten und mit einer Einmalspritze versehenen Betätigungsvorrichtung vor der Betätigung und

Fig. 3    nach der Betätigung,

Fig. 4    einen Halbschnitt durch die Gehäuseverlängerung,

Fig. 5    eine Draufsicht auf die Gehäuseverlängerung,

Fig. 6    einen Längsschnitt durch die Vorrichtung vor dem Spannen des Kolbens und ohne Gehäuseverlängerung,

Fig. 7    eine der Fig. 6 entsprechende Darstellung bei gespannten Kolben.

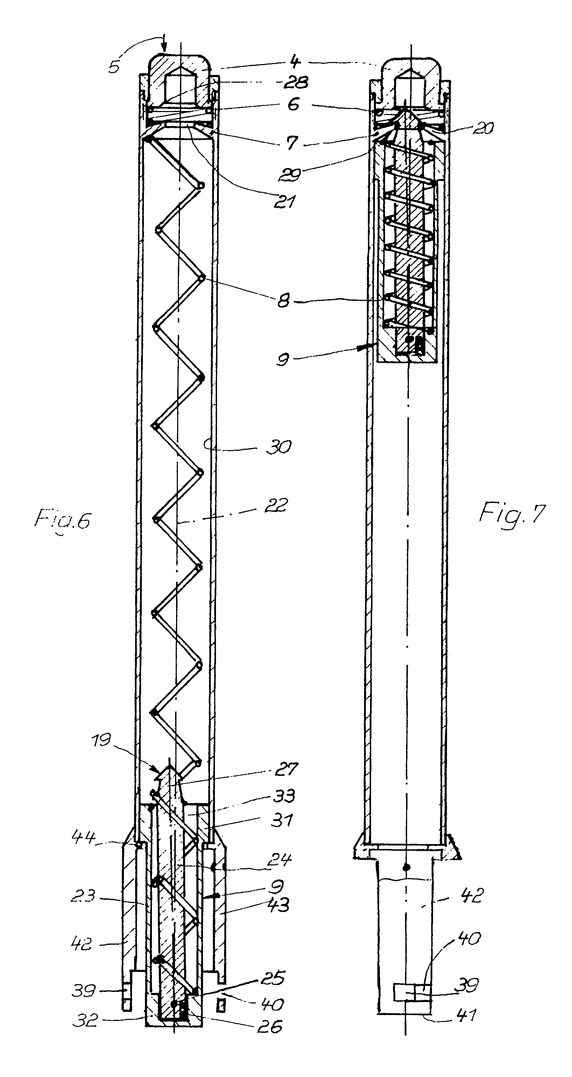

Die Betätigungsvorrichtung besitzt ein rohrförmiges Vorrichtungsgehäuse 1 und eine ebenfalls rohrförmig ausgebildete Gehäuseverlängerung 2. Beide sind über einen Bajonettverschluß 3 kuppelbar. Am freien Ende des Vorrichtungsgehäuses 1 befindet sich das als federbelastete Drucktaste ausgebildete Betätigungsorgan 4, das im Sinne des Pfeils 5 gegen den Widerstand einer Rückstellfeder 6 niedergedrückt werden kann. Diese stützt sich einenends an einem Außenbund des Betätigungsorgans 4 und andernends an einem Innenbund 7 des Vorrichtungsgehäuses 1 ab. Gemäß Fig. 6 liegt an der Unterseite des Innenbunds 7 das obere Ende einer Belastungsfeder 8 eines Vorrichtungskolbens 9 an.

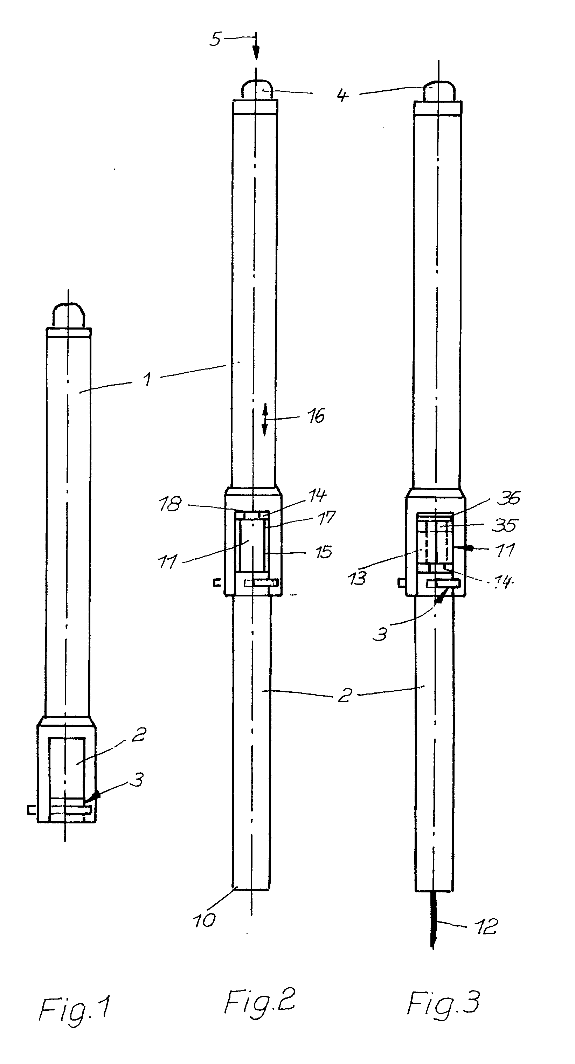

Aus den Fign. 1 und 2 ersieht man, daß die Gehäuseverlängerung 2 in einer um 180° Grad gewendeten Stellung in das Vorrichtungsgehäuse 1 eingeschoben und auch in dieser Stellung mit Hilfe des Bajonettverschlusses 3 festgehalten werden kann. Dabei wird die Belastungsfeder 8 über den Vorrichtungskolben 9 etwas zusammengedrückt, wobei die Feder allerdings weniger gespannt ist als in Fig. 7. Der Kolben nimmt infolgedessen auch eine zwischen den beiden Endstellungen der Fign. 6 und 7 gelegene Zwischenstellung ein. Die gesamte Vorrichtung läßt sich für den Transport auf nahezu die Hälfte verkürzen.

Die in Arbeitstellung anmontierte Gehäuseverlängerung 2 umschließt vor dem Auslösen der Vorrichtung die Einmalspritze 11 vollständig. Dabei steht die Nadel 12 der Spritze über das freie oder Aufsetzende 10 der Gehäuseverlängerung nicht vor.

Die Nadel 12 der Einmalspritze 11 ist in nicht dargestellter aber bekannter Weise mit Hilfe einer Nadelschutzkappe vollständig übergriffen. Außerdem ist der Zylinder 13 der Einmalspritze 11 an seinem von der Nadel 12 abgewandten Ende mit zwei radial vorstehenden, um 180° versetzten Grifflappen 14 ausgestattet. Deren Enden ragen durch je ein Durchtrittsfenster 15 der Vorrichtung nach außen, so daß man die Einmalspritze in der Vorrichtung im Sinne des Doppelpfeils 16 verschieben bzw. ausrichten kann. Gemäß Fig. 2 schiebt man die Einmalspritze entgegen dem Pfeil 5 so weit nach oben, bis die Grifflappen 14 etwa am oberen Ende 18 des zugeordneten Durchtrittsfensters 15 angekommen sind. Dadurch verhindert man ein Vorstellen der Nadel 12 über den Mündungsrand bzw. das Aufsetzende 10 der Gehäuseverlängerung 2. Die Einmalspritze läßt sich in dieser Stellung gegenüber dem Gehäuse 1, 2 mit Hilfe einer Verrastnase 17 arretieren. Sie ist gemäß Fig. 2 so angebracht, daß sich die Grifflappen 14 zwischen der Verrastnase 17 und dem oberen Ende 18 der Durchtrittsfenster 15 befinden. Die Ausbildung im einzelnen und die Elastizität der Einmalspritze bzw. das vorhandene Spiel reichen aus, um beim Auslösen der Vorrichtung die Grifflappen 14 an der Verrastnase 17 im Sinne des Pfeils 5 vorbeischieben zu können.

Der Kolben 9 ist mit einem Verrastelement 19 ausgestattet (Fig. 6). Es wirkt bei gespannter Belastungsfeder 8 mit einem Verrast-Gegenelement 20 zusammen, welches beim Ausführungsbeispiel durch den Innenbund 7 gebildet wird. Die Durchtrittsbohrung 21 des letzteren ist so gewählt, daß das aus einem Kegel oder Kegelstumpf bestehende Verrastelement 19 hindurchtreten kann, wenn es gegenüber der geometrischen Achse 22 der Vorrichtung in etwa ausgerichtet ist. Gemäß Fig. 6 ist dies normalerweise nicht der Fall.

Der Vorrichtungskolben 9 ist nämlich zweiteilig ausgebildet. Das erste Kolbenteil 23 ist mit dem zweiten Kolbenteil 24 über eine sich in Fig. 6 senkrecht zur Blattebene erstreckende Achse 25 schwenkbar verbunden. Zwischen diese beiden Kolbenteile ist eine Kippfeder 26 geschaltet. Es handelt sich dabei, wie auch bei allen übrigen Federn der Vorrichtung, um eine Schraubendruckfeder. Ihre Wirkungslinie befindet sich seitlich der Drehachse 25 und infolgedessen bewirkt sie ein Verschwenken des zweiten Kolbenteils 24 gegenüber dem ersten Kolbenteil 23 in die aus Fig. 6 ersichtliche Stellung. Die geometrische Achse 27 des zweiten Kolbenteils 24 nimmt dabei gegenüber der geometrischen Achse 22 der Vorrichtung eine Schräglage ein. Es ist leicht einzusehen, daß bei hochgeschobenem Kolben 9 (Fig. 7) das zweite Kolbenteil 24 durch den Innenbund 7 in Verbindung mit seiner konischen Form zunächst in eine zentrische Stellung gezwungen wird und nachfolgend wieder zur Seite kippt, wodurch dann der gewissermaßen verdickte Kopf des Zweiten Kolbenteils 24 den Innenbund 7 hintergreift, wie dies Fig. 7 der Zeichnung zeigt.

Wiederum in Verbindung mit der kegeligen Fläche des Verrastelements 19 erreicht man im Zusammenwirken mit der Innenkante 28 des Betätigungsorgans 4 ein Lösen der Verrastvorrichtung 29, wenn man das Betätigungsorgan 4 in Pfeilrichtung 5 niederdrückt. Anstelle eines Kegels oder Kegelstumpfs kann man auch einen Pyramidenstumpf oder eine Pyramide bzw. eine ähnliche Pfeil- oder Keilform wählen.

Das erste Kolbenteil 23 ist in der Zylinderbohrung 30 des Vorrichtungsgehäuses 1 verschiebbar gelagert. Es besitzt eine topfförmige Gestalt mit einem Außenbund 31 am freien Topfrand. Er übernimmt die Gleitführung in der Zylinderbohrung 30. Die Schwenkachse 25 befindet sich im Bereich des Topfbodens 32. Die Kippfeder 26 stützt sich einenends an diesem Topfboden 32 und andernends an einem Absatz einer Federausnehmung des zweiten Kolbenteils 24 ab. Gemäß Fig. 6 überragt das an seinem freien Ende das Verrastelement 19 tragende zweite Kolbenteil 24 das erste Kolbenteil 23 bzw. dessen Außenbund 31.

In den ringförmigen Spaltraum 33 zwischen den beiden Kolbenteilen 23 und 24 ragt das kolbenseitige Ende der Belastungsfeder 8 des Kolbens 9 hinein. Sie stützt sich am Kolbenboden ab.

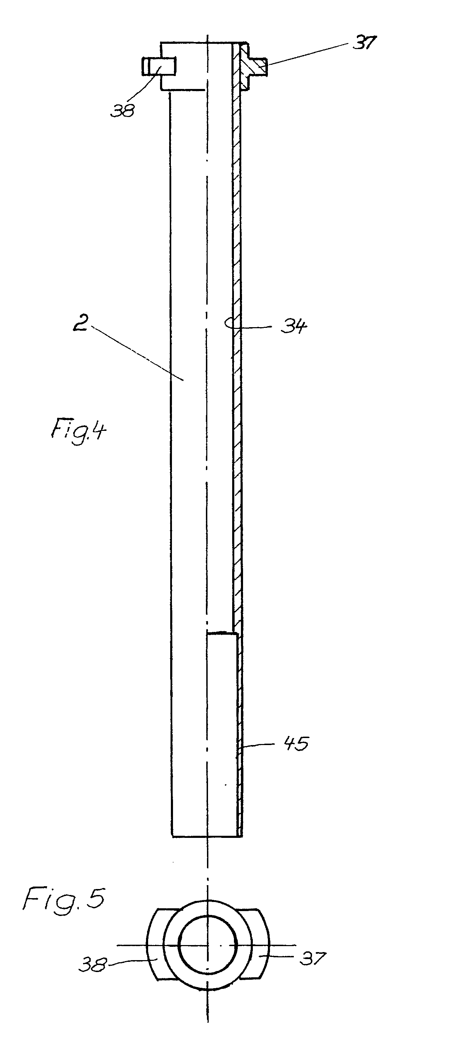

Fig. 7 entnimmt man in Verbindung mit den Fign. 2 und 3, daß sich die Einmalspritze bzw. deren Zylinder 13 im wesentlichen in der Höhlung 34 der Gehäuseverlängerung 2 (Fig. 4) befindet und in die Zylinderbohrung 30 des Vorrichtungsgehäuses 1 vor dem Spritzen das grifflappenseitige Ende des Spritzenzylinders sowie das freie Ende der Kolbenstange 35 der Einmalspritze hineinragen. Üblicherweise ist eine Verdickung 36 am äußeren freien Ende der Kolbenstange 35 vorgesehen, auf welcher der Kolben 9 nach dem Ausrasten auftrifft.

Wenn man die Einmalspritze 11 vor dem Einlegen in die Vorrichtung gegenüber der Darstellung in Fig. 3 um 180° dreht, so kann man mit der die Nadel 12 umhüllenden Nadelschutzkappe den Kolben 9 ins Innere des Vorrichtungsgehäuses 1 hineindrücken bis er schließlich die in Fig. 7 gezeigte Verrast-Endstellung einnimmt. Nach dem Abnehmen dieser Nadelschutzkappe sowie einer üblicherweise auch die Kolbenstange 35 übergreifenden zweiten Schutzkappe wird die Spritze gefüllt und in die Vorrichtung eingelegt. Hernach wird der Bajonettverschluß 3 geschlossen.

Beim Ausführungsbeispiel befinden sich die beiden Bajonettlappen 37 und 38 an der Gehäuseverlängerung 2, während die Bajonettaufnahmen 39 und 40 gemäß Fign. 6 und 7 am unteren Ende des Vorrichtungsgehäuses 1 angebracht sind.

Die Längen des Vorrichtungsgehäuses 1 einerseits und der Gehäuseverlängerung 2 andererseits sind so gewählt, daß bei in der Arbeitsstellung arretiertem Kolben 9 (Fig. 7) die beiden Grifflappen 14 der Spritze 11 das auslöserferne Ende 41 des Vorrichtungsgehäuses 1 noch nicht erreicht haben und in der in Fig. 2 gezeigten Stellung der Spritze 11 die Nadel 12 über den freien Aufsetzrand 10 nicht vorsteht. Es wäre aber auch möglich, daß man beim Spannen des Kolbens 9 bei einem etwas kürzeren Vorrichtungsgehäuse 1 die beiden Grifflappen 14 der Einmalspritze 11 mehr oder weniger tief in die in Achsrichtung randoffenen Durchtrittsfenster 15 des Vorrichtungsgehäuses 1 eintreten läßt. Dem längeren Vorrichtungsgehäuse 1 ist allerdings wegen der leichteren Handhabung der Vorzug zu geben. Die andere Variante bietet demgegenüber den Vorteil einer kürzeren Betätigungsvorrichtung.

Die beiden Durchtrittsfenster 15 sind beim Ausführungsbeispiel im wesentlichen am Vorrichtungsgehäuse 1 ausgebildet, jedoch können sie auch vorwiegend an der Gehäuseverlängerung sein. Am in den Fign. 6 und 7 unteren Ende des Vorrichtungsgehäuse 1 ist die Zylinderwandung zur Bildung der beiden Durchtrittsfenster 15 längsgeschlitzt, so daß nur noch gabelzinkenartige Wandungsteile 42 und 43 stehen bleiben. Am freien Ende des Wandungsteils 42 ist die Bajonettaufnahme 39 und entsprechend am Wandungsteil 43 die Bajonettausnehmung 40 ausgebildet. Das Vorrichtungsgehäuse 1 ist im genannten Bereich zweiteilig ausgebildet, wobei der die Wandungsteile 42 und 43 aufweisende Teil beim Ausführungsbeispiel zweckmäßigerweise einen etwas größeren Außendurchmesser bekommen hat.

Desweiteren entnimmt man Fig. 6, daß der Kolben 9 vor dem die Wandungsteile 42 und 43 aufweisenden Vorrichtungsgehäuseteil montiert werden muß. Ein Innenbund 44 verhindert einerseits das Herausdrücken des Kolbens 9 und er ermöglicht andererseits das Einschieben der Gehäuseverlängerung 2, um in die in Fig. 1 zu sehende Transportstellung zu kommen. Vorzugsweise ist die Wandstarke 45 am lappenfernen Ende etwas reduziert. Weil das lappenferne Ende der Gehäuseverlängerung 2 beim Bilden der Transportstellung und in derselben am Außenbund 31 des ersten Kolbenteils 23 anliegt und die Belastungsfeder 8 bis zum Topfboden 32 reicht, ist die Belastungsfeder 8 in der Transportstellung der Vorrichtung weniger gespannt als in der Auslösestellung gemäß Fig. 7. Dadurch ist in sehr vorteilhafter Weise die Gefahr eines Herausschleuderns der Gehäuseverlängerung 2 aus dem Vorrichtungsgehäuse 1 beim Aufheben der Transportstellung erheblich gemindert.

## Patentansprüche

1. Betätigungsvorrichtung für eine mit einer Nadelschutzkappe sowie zwei Grifflappen (14) versehene Einmalspritze (11) mit einem federbelasteten (8), in einer gespannten Verschiebestellung verrastbaren, mittels eines Betätigungsorgans (4) auslösbaren, in einem Vorrichtungsgehäuse (1) verschiebbaren Vorrichtungskolben (9) mit einem bewegbaren Verrastelement (19), das in der gespannten Verschiebestellung mit einem gehäusefesten Verrast-Gegenelement (20) zusammenwirkt und mit einer abnehmbaren hülsenartigen Gehäuseverlängerung (2), dadurch gekennzeichnet, daß im Bereich der Verbindungsstelle (3) von Vorrichtungsgehäuse (1) und Gehäuseverlängerung (2) zwei um 180° versetzte Durchtrittsfenster (15) für die Grifflappen (14) der Spritze (11) vorhanden sind und die Bohrung der Gehäuseverlängerung (2) eine Aufnahme für zumindest den wesentlichen Teil des Spritzenzylinders (13) bildet, und daß die lichte Weite (13) des Vorrichtungsgehäuses (1) mindestens dem größten Durchmesser der Nadelschutzkappe entspricht und die Länge des Teils des Vorrichtungsgehäuses (1) vom in Arbeitsstellung befindlichen Vorrichtungskolben (9) bis zum auslöserfernen Ende (41) oder gegebenenfalls bis zum inneren Ende der Durchtrittsfenster (15) mindestens der Länge der Spritze (11) vom freien Ende der Nadelschutzkappe bis zu den Grifflappen (14) entspricht.

2. Vorrichtung nach Anspruch 1, dadurch gekennzeichnet, daß der Vorrichtungskolben (9) zweiteilig ausgebildet ist und die beiden Kolbenteile (23, 24) über eine quer zur Kolbenachse (22) verlaufende Schwenkachse (25) miteinander verbunden sind, wobei zwischen die beiden Kolbenteile eine Kippfeder (26) geschaltet ist und eines der Kolbenteile das Verrastelement (19) aufweist.

3. Vorrichtung nach Anspruch 2, dadurch gekennzeichnet, daß das erste Kolbenteil (23) in einer Zylinderbohrung (30) des Vorrichtungsgehäuses (1) verschiebbar gelagert ist und das zweite schwenkbar damit verbundene Kolbenteil (24) das Verrastelement (19) trägt.

4. Vorrichtung nach Anspruch 3, dadurch gekennzeichnet, daß das Verrastelement (19) durch das freie kegel- oder kegelstumpfförmige Ende des zweiten Kolbenteils (24) gebildet ist.

5. Vorrichtung nach Anspruch 3 oder 4, dadurch gekennzeichnet, daß das erste Kolbenteil (23) eine etwa topfförmige Gestalt aufweist, wobei der Topfboden (32) an der Kolbenstange (35) der Spritze (11) anlegbar ist, und daß das zweite Kolbenteil (24) im Bereich des Topfbodens (32) am ersten (23) gelagert ist, wobei sein freies Ende das erste Kolbenteil (23) überragt und die Kippfeder (26) vorzugsweise im Bereich des Topfbodens (32) seitlich der Kolbenachse (27) zwischen die Kolbenteile (23, 24) geschaltet ist.

6. Vorrichtung nach wenigstens einem der Ansprüche 3 bis 5, gekennzeichnet durch ein durch einen Innenbund (7) des Vorrichtungsgehäuses (1) gebildetes Verrast-Gegenelement, wobei die lichte Weite des Innenbunds (7) einen Durchlaß (21) für das etwa zentrisch ausgerichtete Verrastelement (19) bildet.

7. Vorrichtung nach Anspruch 5 oder 6, dadurch gekennzeichnet, daß in einen ringförmigen Spaltraum (33) zwischen den beiden Kolbenteilen (23, 24) das kolbenseitige Ende der Belastungsfeder (8) des Kolbens (9) eingreift.

8. Vorrichtung nach Anspruch 7, dadurch gekennzeichnet, daß sich das kolbenferne Ende der Kolben-Belastungsfeder (8) am Innenbund (7) der Vorrichtung abstützt.

9. Vorrichtung nach wenigstens einem der Ansprüche 3 bis 8, dadurch gekennzeichnet, daß das Betätigungsorgan (4) ein zentrischer, federbelasteter (6) im Vorrichtungsgehäuse (1) begrenzt verschiebbarer Drücker mit zentrischer Höhlung ist, der etwa in seiner eingedrückten Lage den zweiten Kolbenteil (24) in eine Ausraststellung bringt, wobei der freie Rand (28) der Höhlung beim Ausrasten mit dem Verrastelement (19) am zweiten Kolbenteil (24) zusammenwirkt.

10. Vorrichtung nach wenigstens einem der vorhergehenden Ansprüche, dadurch gekennzeichnet, daß das Vorrichtungsgehäuse (1) und die Gehäuseverlängerung (2) über einen Bajonettverschluß (3) kuppelbar sind, wobei sich vorzugsweise die Bajonettlappen (37, 38) an der Gehäuseverlängerung (2) und die Bajonett-Aufnahme (39, 40) am Vorrichtungsgehäuse (1) befinden.

11. Vorrichtung nach wenigstens einem der vorhergehenden Ansprüche, dadurch gekennzeichnet, daß das Vorrichtungsgehäuse (1) an seinem der Gehäuseverlängerung (2) zugekehrten Ende gabelförmig gestaltet ist, wobei die Zwischenräume zwischen den Gabelzinken (42, 43) die Durchtrittsfenster (15) bilden und die Bajonett-Aufnahmen (39, 40) vorzugsweise am Endbereich der Gabelzinken angeordnet sind.

12. Vorrichtung nach wenigstens einem der vorhergehenden Ansprüche, dadurch gekennzeichnet, daß der Abstand eines Außenbunds (31) des gespannten Vorrichtungskolbens (9) vom auslöserfernen Ende (41) des Vorrichtungsgehäuses (1) mindestens der Länge der Gehäuseverlängerung (2) entspricht.

13. Vorrichtung nach wenigstens einem der vorhergehenden Ansprüche, gekennzeichnet durch wenigstens eine Verrastnase (17) od.dgl. im Bereich des gegen das Betätigungsorgan (4) weisenden Endes der Durchtrittsfenster (15) zum elastisch ausrastbaren Festhalten des Spritzenzylinders (13) in einer zurückgeschobenen Spritzenstellung.

14. Vorrichtung nach wenigstens einem der Ansprüche 3 bis 13, dadurch gekennzeichnet, daß das erste Kolbenteil (23) an seinem vom Topfboden (32) entfernten freien Ende einen äußeren Führungsbund (31) trägt und der ringförmige Spaltraum zwischen dem bundfreien Teilstück des ersten Kolbenteils (23) und einem Innenbund (44) des Vorrichtungsgehäuses (1) einen Durchtritt für die Gehäuseverlängerung (2) in der Transportstellung der Vorrichtung bildet.

Fig.1            Fig.2            Fig.3

Fig.4

Fig.5

Fig.6

Fig.7